(19) Europäisches Patentamt
European Patent Office
Office européen des brevets

(11) **EP 3 011 895 B1**

## (12) EUROPEAN PATENT SPECIFICATION

(45) Date of publication and mention
of the grant of the patent:
**11.08.2021 Bulletin 2021/32**

(21) Application number: **15155659.4**

(22) Date of filing: **18.02.2015**

(51) Int Cl.:
*A61B 5/369* (2021.01)     *A61B 5/00* (2006.01)
*A61B 5/16* (2006.01)      *G16H 50/20* (2018.01)

(54) **Determining cognitive load of a subject from electroencephalography (EEG) signals**

Bestimmung der kognitiven Belastung eines Subjekts aus Elektroenzephalografie- (EEG)-Signalen

Détermination de la charge cognitive d'un sujet à partir d'électroencéphalographie (EEG) des signaux

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2014 IN 3390MU2014**

(43) Date of publication of application:
**27.04.2016 Bulletin 2016/17**

(73) Proprietor: **Tata Consultancy Services Limited Maharashtra (IN)**

(72) Inventors:
- **Sinharay, Arijit**
  **700156 West Bengal (IN)**
- **Chatterjee, Debatri**
  **700156 West Bengal (IN)**
- **Pal, Arpan**
  **700156 West Bengal (IN)**

(74) Representative: **Potter Clarkson
The Belgrave Centre
Talbot Street
Nottingham NG1 5GG (GB)**

(56) References cited:
EP-A2- 2 200 347      CN-B- 102 063 180
US-A1- 2007 185 697

- **BESSERVE ET AL: "Prediction of performance level during a cognitive task from ongoing EEG oscillatory activities", CLINICAL NEUROPHYSIOLOGY, ELSEVIER SCIENCE, IE, vol. 119, no. 4, 4 March 2008 (2008-03-04) , pages 897-908, XP022524723, ISSN: 1388-2457, DOI: 10.1016/J.CLINPH.2007.12.003**
- **A Gevins: "High-resolution EEG mapping of cortical activation related to working memory: effects of task difficulty, type of processing, and practice", Cerebral Cortex, vol. 7, no. 4, 1 June 1997 (1997-06-01), pages 374-385, XP055493458, DOI: 10.1093/cercor/7.4.374**
- **CHATTERJEE DEBATRI ET AL: "EEG-based fuzzy cognitive load classification during logical analysis of program segments", 2013 IEEE INTERNATIONAL CONFERENCE ON FUZZY SYSTEMS (FUZZ-IEEE), IEEE, 7 July 2013 (2013-07-07), pages 1-6, XP032496146, ISSN: 1098-7584, DOI: 10.1109/FUZZ-IEEE.2013.6622508 [retrieved on 2013-10-04]**

EP 3 011 895 B1

**Description**

CROSS-REFERENCE TO RELATED APPLICATIONS AND PRIORITY

The present application does claim priority from an Indian patent application number 3390/MUM/2014 filed on 26th October, 2014.

TECHNICAL FIELD

The present subject matter described herein, in general, relates to processing of bioelectric signals for diagnostic purposes, and more particularly to determining a cognitive load of a subject from Electroencephalography (EEG) signals.

BACKGROUND

Cognitive load is a total amount of mental activity imposed on a memory of a subject/human while performing any cognitive task. High cognitive load may significantly influence performance of the subject leading to poor outcome, stress, or anxiety. Now days, measurement of the cognitive load is receiving increased attention. Physiological measures like brain signals, a galvanic skin response, a functional magnetic resonance imaging (MRI), and an electroencephalogram (EEG) technique may be used to quantify the cognitive load. As compared to other available techniques, the EEG technique is relatively in-expensive, non-invasive and has excellent temporal resolution. Currently a time-domain and a frequency-domain of the EEG signals and statistical parameters have been used to measure the cognitive load.

There are various devices available in market to measure the cognitive load using the EEG technique. One type of EEG devices may include high cost and high resolution EEG devices which may fall under precise medical diagnostic devices and other type may include low cost low resolution EEG devices used for all-purpose diagnostic services. The low cost low resolution EEG devices come with lower number of EEG channels, hence may miss the EEG channels that are sensitive to the cognitive load. Further, sensitive positions of the EEG channels may be subjective and may vary from person to person. Moreover, the low cost low resolution EEG devices do not come with extra channels (EOG) to measure and remove a noise contaminating the EEG signals. The noise may pose serious issue in accurately measuring the cognitive load of the subject.

United States patent US 2007/185697 A1 discloses a method for classifying brain states using EEG signals collected from sensors attached to the scalps of users. The method is capable of determining the persistence of brain states and the transitions between brain states wherein the brain states are associated with at least one condition chosen from the group of user conditions comprising: interruptability, cognitive Workload, task engagement, communication mediation, interpreting and predicting system response, surprise, satisfaction, and frustration. The method comprises building a classifier model using labeled EEG data and classifying brain states in unlabeled EEG data using the classifier model. The classifier model is built by determining brain states; collecting a set of labeled EEG data; and dividing the set into overlapping time windows. The time dimension is removed from each time window, features are generated for the time windows, and the classifier model is built. Features are generated from the time windows of the labeled EEG data by computing the base features of the time windows; combining the base features to form a larger feature set; pruning the larger feature set without regard to machine learning techniques; and further pruning the feature set for a particular machine learning technique. Brain states are classified using the classifier model by collecting a set of unlabeled EEG data, dividing the unlabeled EEG data into overlapping time windows, and removing the time dimension from each time window. Further, features required by the classifier model are generated from the time windows. The classifier model is used to classify brain states in the unlabeled EEG data. The size of averaging kernels is chosen and the classifier output is averaged over adjacent time windows. The labeled and unlabeled EG data contains both brain state data and artifacts wherein the artifacts may be blocked by filtering.

CN 102063180B discloses a Hilbert-Huang Transform (HHT)-based high-frequency combined coding steady state visual evoked potential brain-computer interface method, which comprises the following steps of: connecting hardware, expressing different targets by using nn stimulation sequences formed by time sequence permutation and combination through n high-frequency stimulation frequencies, coding the stimulation sequences to form a code base, then executing electroencephalogram data characteristics by adopting a Hilbert-Huang Transform (HHT)-based variable frequency electroencephalogram signal characteristic extraction method, acquiring a Hilbert time-frequency graph of the electroencephalogram data, and comparing the extracted electroencephalogram data characteristics with the code base by using a local frequency spectrum extreme value target identification method to realize quantified target identification accuracy.

The disclosure of European Patent EP 2200347A2 relates to customization of a hearing instrument. It discloses a method for operating a hearing instrument for processing an input sound and to provide an output stimulus according to a user's particular needs and comprises the steps a) providing an estimate of the present cognitive load of the user;

b) providing processing of an input signal originating from the input sound according to a user's particular needs; and c) adapting the processing in dependence of the estimate the present cognitive load of the user. This has the advantage that the functionality of the hearing aid system is adapted to the current mental state of the user. The estimate of the present cognitive load of a user is produced by in-situ direct measures of cognitive load (e.g. based on EEG-measurements etc.) or by an on-line cognitive model in the hearing aid system whose parameters have been preferably adjusted to fit to the individual user. An estimate of a user's cognitive status or cognitive load can e.g. be based on an estimate of the user's working memory capacity.

Besserve, M. et al. "Prediction of performance level during a cognitive task from ongoing EEG oscillatory activities", Clinical Neurophysiology, Vol. 119(4) pp 897-908 relates to tracking the level of performance in cognitive tasks. A methodology is presented that combines various ongoing EEG measurements to predict performance level during a cognitive task. A voting approach is proposed that combines the outputs of elementary support vector machine (SVM) classifiers derived from various sets of EEG parameters in different frequency bands. The spectral power and phase synchrony of the oscillatory activities are used to classify the periods of rapid reaction time (RT) versus the slow RT responses of each subject.

Gevins, A. "High-resolution EEG mapping of cortical activation related to working memory: effects of task difficulty, type of processing, and practice", Cerebral Cortex, Vol. 7(4), pp 374-385 describes changes in cortical activity during working memory tasks were examined with electroencephalograms (EEGs) sampled from 115 channels and spatially sharpened with magnetic resonance imaging (MRI)-based finite element deblurring. Eight subjects performed tasks requiring comparison of each stimulus to a preceding one on verbal or spatial attributes. A frontal midline theta rhythm increased in magnitude with increased memory load. Dipole models localized this signal to the region of the anterior cingulate cortex. A slow (low-frequency), parietocentral, alpha signal decreased with increased working memory load. These signals were insensitive to the type of stimulus attribute being processed. A faster (higher-frequency), occipitoparietal, alpha signal was relatively attenuated in the spatial version of the task, especially over the posterior right hemisphere. CHATTERJEE DEBATRI ET AL, "EEG-based fuzzy cognitive load classification during logical analysis of program segments", 2013 IEEE INTERNATIONAL CONFERENCE ON FUZZY SYSTEMS (FUZZ-IEEE) relates to a novel scheme for cognitive load classification of subjects engaged in program analysis using EEG signals selected from the channels AF3, F3, FC5, F7.

Different algorithmic approaches are available in prior art to measure the cognitive load of the subject. However these algorithms come with excessive computation and also fail to provide accurate results in measurement of the cognitive load, particularly in measurement of the cognitive load with low cost, low resolution EEG devices.

SUMMARY

This summary is provided to introduce aspects related to systems and methods for determining a cognitive load of a subject from Electroencephalography (EEG) signals and the aspects are further described below in the detailed description. This summary is not intended to identify essential features of the claimed subject matter nor is it intended for use in determining or limiting the scope of the claimed subject matter; the invention being defined by the appended claims.

In one implementation, a method for determining a cognitive load of a subject from Electroencephalography (EEG) signals is disclosed. The method comprises receiving, by a processor, the EEG signals from a set of EEG channels associated with a left-frontal brain lobe. The EEG signals are received from a low resolution EEG device comprising a maximum of fourteen EEG channels. The EEG signals are associated with the subject performing a cognitive task. The method further comprises preprocessing, by the processor, the EEG signals using a Hilbert-Huang Transform (HHT) filter to remove a noise corresponding to one or more unrelated, non-cerebral artifacts to generate preprocessed EEG signals. The method further comprises extracting, by the processor, features comprising Fast Fourier Transform (FFT) based alpha and theta band power, from the preprocessed EEG signals. The method further comprises generating, by the processor, a feature vector from the features. The method further comprises classifying, by the processor, the feature vector using a supervised machine learning technique to determine the cognitive load of the subject.

In one implementation, a system for determining a cognitive load of a subject from Electroencephalography (EEG) signals is disclosed. The system comprises a processor and a memory coupled to the processor. The processor is capable of executing programmed instructions stored in the memory to receive the EEG signals from a set of EEG channels associated with a left-frontal brain lobe. The EEG signals are associated with the subject performing a cognitive task. The low resolution EEG device is linked with a maximum of fourteen EEG channels. The processor further preprocesses the EEG signals using a Hilbert-Huang Transform (HHT) filter that decomposes the EEG signals into empirical mode signals and remove noise corresponding to one or more unrelated, non-cerebral artifacts by replacing frequency of the EEG signals with a median value at a time point when the frequency of the EEG signals overshoots a predefined level at the time point, to generate preprocessed EEG signals. The non-cerebral artifacts are originated from body parts and outside body parts, movement of the subject, settling of EEG electrodes, spikes originating from a momentary change in an impedance of the EEG electrode. The processor further extracts features comprising Fast Fourier Transform (FFT) based alpha and theta band power, from the preprocessed EEG signals. The processor further generates a feature vector from the features. The processor further classifies the feature vector using a supervised machine learning technique to determine the cognitive load of the subject.

BRIEF DESCRIPTION OF THE DRAWINGS

The detailed description is described with reference to the accompanying figures. In the figures, the left-most digit(s) of a reference number identifies the figure in which the reference number first appears. The same numbers are used throughout the drawings to refer like features and components.

Figure 1 illustrates a network implementation of a system for determining a cognitive load of a subject from Electroencephalography (EEG) signals, in accordance with an embodiment of the present subject matter.

Figure 2 illustrates a placement of 14 EEG channels of the low resolution EEG device- 'Emotiv™' headset, in accordance with an exemplary embodiment of the present subject matter.

Figure 3 illustrates variety of paths for processing the EEG signals with different algorithmic approaches, in accordance with an embodiment of the present subject matter.

Figure 4 illustrates one-way Analysis of Variance (ANOVA) test (Box plot) results given in Table 2 containing cognitive scores for executing cognitive tasks of a low cognitive load (L) and a high cognitive load (H), in accordance with an embodiment of the present subject matter.

Figure 5 illustrates an investigation of usefulness of a Hilbert-Huang Transform (HHT) filter in processing of the EEG signals to determine the cognitive load of the subject, in accordance with an embodiment of the present subject matter.

Figure 6 illustrates a method for determining a cognitive load of a subject from Electroencephalography (EEG) signals, in accordance with an embodiment of the present subject matter.

DETAILED DESCRIPTION

Systems and methods for determining a cognitive load of a subject from Electroencephalography (EEG) signals are described. The EEG signals may be received from a set of EEG channels. The EEG signals may be associated with the subject performing a cognitive task. The EEG signals may be preprocessed to remove noise corresponding to one or more unrelated, non-cerebral artifacts to generate preprocessed EEG signals. Features comprising Fast Fourier Transform (FFT) based alpha and theta band power may be extracted from the preprocessed EEG signals. A feature vector may be generated from the features. The feature vector may be classified using a supervised machine learning technique to determine the cognitive load of the subject.

While aspects of described system and method for determining a cognitive load of a subject from Electroencephalography (EEG) signals may be implemented in any number of different computing systems, environments, and/or configurations, the embodiments are described in the context of the following exemplary system.

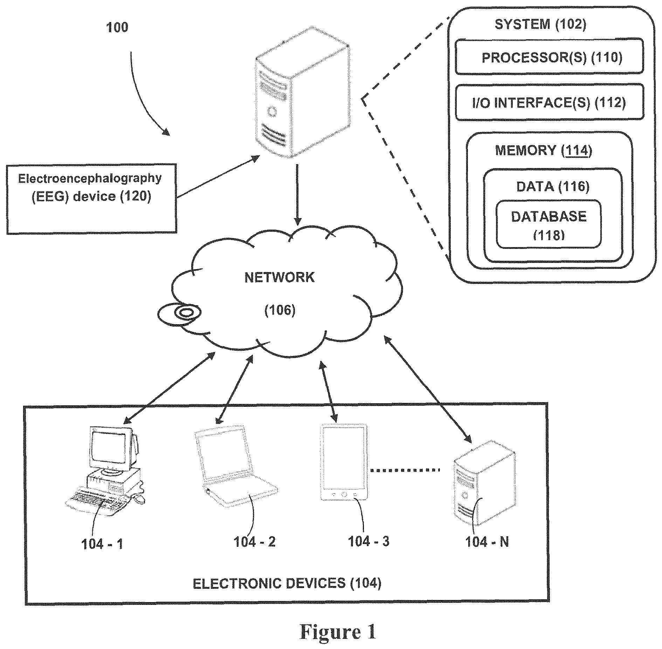

Referring now to Figure 1, a network implementation 100 of a system 102 for determining a cognitive load of a subject from Electroencephalography (EEG) signals is illustrated, in accordance with an embodiment of the present subject matter. The system 102 may determine the cognitive load of the subject from the EEG signals received from an EEG device 120. The EEG device is a low resolution EEG device. The EEG device is linked to a plurality of EEG channels. The low resolution EEG device may be linked with maximum of fourteen channels. In one embodiment, the system 102 may receive the EEG signals from a set of EEG channels associated with a left-frontal brain lobe. The EEG signals may be associated with the subject performing a cognitive task. Post receiving the EEG signals, the system preprocesses the EEG signals to remove noise corresponding to one or more non-cerebral artifacts from the EEG signals to generate preprocessed EEG signals. The system preprocesses the EEG signals using a Hilbert-Huang Transform (HHT) filter to remove the noise corresponding to one or more non-cerebral artifacts to generate the preprocessed EEG signals.

The system extracts features comprising Fast Fourier Transform (FFT) based alpha and theta band power from the preprocessed EEG signals. Subsequent to extracting the features, the system generates a feature vector from the features. Post generating the feature vector, the system classifies the feature vector using a supervised machine learning technique to determine the cognitive load of the subject.

Although the present subject matter is explained considering that the system 102 is implemented on a server, it may be understood that the system 102 may also be implemented in a variety of computing systems, such as a laptop computer, a desktop computer, a notebook, a workstation, a mainframe computer, a server, a network server, and the like. In one implementation, the system 102 may be implemented in a cloud-based environment. It will be understood that the system 102 may be accessed by multiple users through one or more user devices 104-1, 104-2... 104-N, collectively referred to as user devices 104 hereinafter, or applications residing on the user devices 104. Examples of the user devices 104 may include, but are not limited to, a portable computer, a personal digital assistant, a handheld device, a server and a workstation. The user devices 104 are communicatively coupled to the system 102 through a network 106.

In one implementation, the network 106 may be a wireless network, a wired network or a combination thereof. The network 106 can be implemented as one of the different types of networks, such as intranet, local area network (LAN), wide area network (WAN), the internet, and the like. The network 106 may either be a dedicated network or a shared network. The shared network represents an association of the different types of networks that use a variety of protocols, for example, Hypertext Transfer Protocol (HTTP), Transmission Control Protocol/Internet Protocol (TCP/IP), Wireless Application Protocol (WAP), and the like, to communicate with one another. Further the network 106 may include a variety of network devices, including routers, bridges, servers, computing devices, storage devices, and the like.

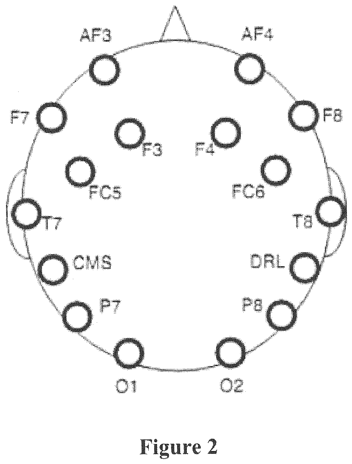

Referring now to Figure 2, the system 102 is illustrated in accordance with an embodiment of the present subject matter. In one embodiment, the system 102 may include at least one processor 110, an input/output (I/O) interface 112, and a memory 114. The at least one processor 110 may be implemented as one or more microprocessors, microcomputers, microcontrollers, digital signal processors, central processing units, state machines, logic circuitries, and/or any devices that manipulate signals based on operational instructions. Among other capabilities, the at least one processor 110 is configured to fetch and execute computer-readable instructions stored in the memory 114.

The I/O interface 112 may include a variety of software and hardware interfaces, for example, a web interface, a graphical user interface, and the like. The I/O interface 112 may allow the system 102 to interact with a user directly or through the client devices 104. Further, the I/O interface 112 may enable the system 102 to communicate with other computing devices, such as web servers and external data servers (not shown). The I/O interface 112 can facilitate multiple communications within a wide variety of networks and protocol types, including wired networks, for example, LAN, cable, etc., and wireless networks, such as WLAN, cellular, or satellite. The I/O interface 112 may include one or more ports for connecting a number of devices to one another or to another server.

The memory 114 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. The memory 114 may include the programmed instructions and data 116.

The data 116, amongst other things, serves as a repository for storing data processed, received, and generated by execution of the programmed instructions. The data 116 may also include a system database 118.

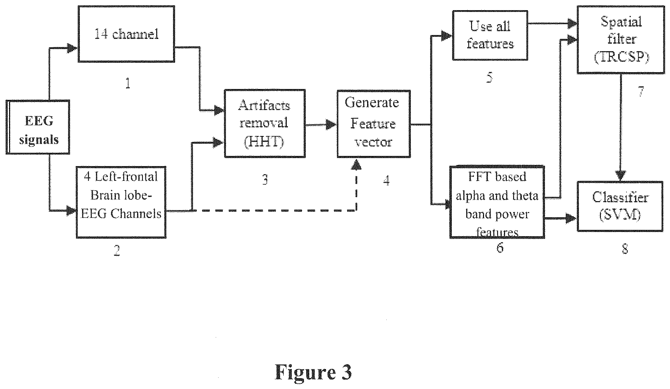

In one implementation, at first, a user may use the client device 104 to access the system 102 via the I/O interface 112. The user may register using the I/O interface 112 in order to use the system 102. The working of the system 102 may be explained in detail in Figures 2 and 3 explained below. The system 102 may be used for determining a cognitive load of a subject from Electroencephalography (EEG) signals.

In order to determine the cognitive load of the subject, the system 102 receives the EEG signals from a set of EEG channels linked to an EEG device 120. The EEG signals are associated with the subject performing a cognitive task. The cognitive task may be a task in which a mental activity is imposed on a memory of the subject. For example, the cognitive task may comprise a problem solving task, a decision making task, language skills, and the like. The EEG signals are received from low resolution EEG electrodes/channels. The low resolution EEG device comprises a maximum of fourteen EEG channels.

Cortex or cerebrum region is the largest part of a brain. The cortex or the cerebrum region is associated with different functions of the brain like critical thinking, perception, decision making and the like. Different lobes of the cortex are responsible for different cognitive functions of the brain. For example, an occipital lobe is associated with visual perception, a temporal lobe is associated with perception and recognition of an auditory stimuli and the like. The cognitive load variations for tasks of different difficulty levels are most clearly visible if frontal and parietal lobes are considered. The system 102 receives the EEG signals from the set of EEG channels associated with a left-frontal brain lobe. The set of EEG channels may comprise four EEG channels associated with the left-frontal brain lobe. Frontal region of the brain is mainly responsible for cognitive tasks like problem solving or decision making and the like. By way of an example, subjects (participants) selected in the present experiments are right-handed human beings and hence their left-frontal brain lobe is most indicative of the cognitive load. Further, the low resolution device used in the present disclosure has only four (AF3, F7, F3 and FC5) channels/electrodes in the left-frontal brain lobe region. Hence, the set of EEG channels comprises four channels (AF3, F7, F3 and FC5) associated with the left-frontal brain lobe. For example, the combination of the four channels (AF3, F7, F3 and FC5) associated with the left-frontal brain lobe is proved as a promising combination for determining the cognitive load of the subject.

Post receiving the EEG signals, the system 102 preprocesses the EEG signals to generate preprocessed EEG signals. The system 102 preprocesses the EEG signals to remove a noise corresponding to one or more non-cerebral artifacts to generate the preprocessed EEG signals. The system preprocesses the EEG signals using a Hilbert-Huang Transform (HHT) filter. The EEG signals may be susceptible to the one or more unrelated, non-cerebral artifacts. The EEG signals may be contaminated by the one or more non-cerebral artifacts. The one or more non-cerebral artifacts may comprise artifacts not directly associated with brain activity and may be associated with other body parts. The one or more non-cerebral artifacts may comprise eye-blinks, eye movements, extra-ocular muscle activity, facial muscle movements, cardiac artifacts, and the like. The one or more non-cerebral artifacts may also comprise artifacts originated from outside the body of the subject and may be associated with a machine or an environment. The one or more non-cerebral artifacts may also comprise movement of the subject, settling of EEG electrodes, spikes originating from a momentary change in an impedance of an EEG electrode, or poor grounding of the EEG electrodes.

The Hilbert Huang Transform (HHT) filter decomposes the EEG signals into empirical mode signals. The empirical mode signals can be used to visualize changes in the EEG signals in both a time domain and a frequency domain, Hence the Hilbert Huang Transform (HHT) filter enable manipulation of a frequency of the EEG signals at a given time point. Further, Hilbert Huang Transform (HHT) also provides computational simplicity over other time frequency decomposition filters like wavelet transform and the like. When at the given time point, the frequency of the EEG signals overshoots a predefined level, the Hilbert Huang Transform (HHT) filter adaptively removes the noise from the EEG signals using simple statistical measures, in the context of the claimed invention, replacing the frequency with a median value at the given time point.

Subsequent to generating the preprocessed EEG signals, the system 102 extracts features from the preprocessed EEG signals using techniques known in the art. The features are extracted based upon experimentation and quality of results there from. According to the claimed invention, the system 102 extracts features comprising Fast Fourier Transform (FFT) based alpha and theta band power from the preprocessed EEG signals.

Post extracting the features, the system generates a feature vector from the features. Depending on an algorithmic approach used, a number of features, in different combination are used to generate the feature vector. Referring to Table 1, different algorithmic approaches, comprising various combinations of features, in path1 to path6 are explained. The features comprises a variance, Hjorth parameters, alpha ($\alpha$), beta ($\beta$), theta ($\theta$), delta ($\delta$), gamma (y) band powers, band power ratios such as $\beta/\theta$ and $\alpha/\delta$, and the like.

According to the claimed invention, the feature vector is generated from the features comprising the Fast Fourier Transform (FFT) based alpha and theta band power features. The selection of the Fast Fourier Transform (FFT) based alpha and theta band power features eliminate need of spatial filtration and provides sufficient quality of the features to further measure cognitive load of the subject. Since the selected features comprises only two types of features such as FFT based alpha and theta band power features, there is no need of spatial filtration. Thus by reducing the use of spatial filtration, the computation complexity in processing of the system 102 and a method 600 is considerably reduced.

Post generating the feature vector, the system classifies the feature vector using a supervised machine learning technique. The system classifies the feature vector using a supervised machine learning technique to determine the cognitive load of the subject. The supervised machine learning technique may be a Support Vector Machine (SVM) classifier.

According to an exemplary embodiment, determination of a cognitive load of a subject from Electroencephalography (EEG) signals is explained below. Experimental work and data collected during the experiments is also provided. Specifically, a group of 10 participants (subjects), aged between 25-30yrs, are selected. All the 10 participants are right-handed male and have English as second language. The selection of the 10 participants ensures minimum variance in level of expertise and brain lateralization across all the 10 participants. Each participant is connected with a low resolution EEG device - 'Emotiv™' headset positioned on head. The low resolution EEG device - 'Emotiv™' headset has fourteen EEG channels. The fourteen EEG channels of 'Emotiv™' headset are arranged on head of the participant to probe different brain lobes. According to an exemplary embodiment, placement of 14 EEG channels of the low resolution EEG device- 'Emotiv™' headset is shown in Figure 2.

In the experimental work, 10 participants are given with two sets of stimuli to work with. The two sets of stimuli are presented on an about 24.6 cm (9.7-inch) iPad™. The two sets of stimuli contain two high cognitive load tasks and two low cognitive load tasks. EEG signals corresponding to first set of stimuli are used as a training data and the second set of stimuli are used as a test data and vice versa. An average of results received from the two sets of stimuli is used as a final result.

The two high cognitive load tasks and two low cognitive load tasks are pertaining to reading activity. For a low cognitive load task, the subjects are asked to mentally count a number of two letter words (except 'of') while reading an English passage and report the number of counted words at the end. For a high cognitive load task, the subjects are asked to count two-letter words as well as three-letter words separately (except 'of' and 'the') while reading a similar English passage and report the number of counted words at the end.

While executing the two high cognitive load tasks and two low cognitive load tasks on each of the 10 participants, the EEG signals are received by the system 102 from the 'Emotiv™' device. As shown in Figure 3 and Table 2, four trials are executed for processing the EEG signals. The three trials as shown in Table 1, with different algorithmic approaches corresponding to path1 to path6, are selected for processing the EEG signals. The details of the different algorithmic approaches as adopted are given in Table 1 and Figure 3. Numbers provided in the path1 to path6, shown in Table 1, are referred to signal processing blocks shown in Figure 3.

The features are extracted from the EEG signals based on the path selected from path1 to path6. A feature vector is generated from the features extracted from the EEG signals. The feature vector may comprise variance, Hjorth parameters, alpha ($\delta$), beta ($\beta$), theta ($\theta$), delta ($\delta$), gamma ($\gamma$) band powers and ratios of band powers $\beta/\theta$ and $\alpha/\delta$ based on selection of the path from Path1 to Path6.

Table 1: Details of different Algorithm approaches adopted

| Motivation | Algorithm chain | Approach used |
|---|---|---|
| **Trial 1:**<br>Choice of brain lobe to be probed :- To examine if a subset of all EEG channels can be used with compromising on accuracy | Path1:<br>$1 \rightarrow 3 \rightarrow 4 \rightarrow 5 \rightarrow 7 \rightarrow 8$ | i) Probing all the brain lobes using all 14 EEG channels of EEG device-Emotive headset<br>ii) Using full feature vector set comprising all the EEG channels (14 EEG channels shown in Figure 2)<br>iii) Using Tikhonov-Regularized Common Spatial Pattern - Spatial Filter (TRCSP) on the feature vector set<br>iv) Using Support Vector Machine (SVM) for final classification on the feature vector set |
| | Path2:<br>$2 \rightarrow 3 \rightarrow 4 \rightarrow 5 \rightarrow 7 \rightarrow 8$ | i) Probing only left-frontal brain (AF3, F7, F3, FC5)<br><br>ii) Using full feature set on above (AF3, F7, F3, FC5) EEG channels<br>iii) Using TRCSP on the feature vector set<br>iv) SVM for final classification |
| **Trial 2:**<br>Choice of features to be used :- to examine if a reduced feature set can lead to same accuracy | Path3:<br>$1 \rightarrow 3 \rightarrow 4 \rightarrow 6 \rightarrow 7 \rightarrow 8$ | i) Probing all the brain lobes<br>ii) Using only alpha and theta band power features<br>iii) This alpha and theta band power features set fed to TRCSP<br>iv) SVM for final classification |
| | Path4:<br>$2 \rightarrow 3 \rightarrow 4 \rightarrow 6 \rightarrow 7 \rightarrow 8$ | i) Probing only left-frontal brain (AF3, F7, F3, FC5)<br><br>ii) Using only alpha and theta band power features<br>iii) Alpha and theta band power feature set fed to TRCSP<br>iv) SVM for final classification |
| **Trial 3:** | Path 5: | i) Probing all the brain lobes |

(continued)

| Motivation | Algorithm chain | Approach used |
|---|---|---|
| Use of spatial filters: - to examine the need of CSP as 'Emotiv™' does not have neighboring electrode in true sense | 1→3→4→6→8 | ii) Using only alpha and theta band power features<br>iii) Alpha and theta band power features set fed to SVM |
| | Path 6:<br>2→3→4→6→8<br><br>(Most preferred path) | i) Probing only left-frontal brain (AF3, F7, F3, FC5)<br>ii) Using only alpha and theta band power features iii) Alpha and theta band power features set fed to SVM |
| Trial 4:<br>Effect of HHT:-Eye-blink lies in (0.4-4 Hz) and features in (4-12 Hz). So, tried to examine usefulness of artifacts removal | Preferred path with HHT | i) Selected preferred path from Path1 through Path6 |
| | Preferred path without HHT | ii) Selected Path1 through Path6 but without using HHT |

Referring to Figure 3 and Table 1, the different algorithm approaches are compared based on i) cognitive score (CS) and ii) computational complexity (CC). The i) cognitive score (CS) and the ii) computational complexity (CC) is obtained while classifying the EEG signals following a particular signal processing path. The cognitive score (CS) is defined in Equation (1) provided below. Features extracted from the training data are used to train Support Vector Machine (SVM). The features are selected features or output of TRCSP, based on the selected path. Same features are calculated from the test data as well, and the features are further fed to SVM. The analysis is done in a non-overlapping window (5 Sec.) basis. The non-overlapping window (5 Sec.) basis comprises sub dividing the preprocessed EEG signal into a number of windows of length 5sec. each.

Finally, a cognitive score for a particular path for each participant is calculated using Equation (1). The cognitive score (CS) represents the cognitive load of the subject.

$$CS = \frac{\sum m_i \times w_i}{n} \ldots\ldots\ldots\ldots \text{Equation (1)}$$

In Equation (1), $m_i$ is number of windows reported as class $i$, $n$ is total number of windows in a test trial and $w_i$ is a weight-factor. For high cognitive load class $w_i$ =100 and for low cognitive load class $w_i$ =0. Hence for trial containing low cognitive load task $CS \approx 0$ and for trial containing high cognitive load task $CS \approx 100$. The computational complexity (CC) of an algorithm is number of processing steps required for a particular input. In present disclosure, the computational complexity (CC) is defined in Equation (2) as shown below.

$$CC = n_c \times (L + C_{HHT}) + n_c \times (m_f \times F) + (n_c \times m_f) \times C_{CSP} \ldots\ldots\ldots\ldots \text{Equation (2)}$$

In Equation (2), $n_c$ is a number of channels selected, $L$ is a computational complexity for a single channel, $C_{HHT}$ is the computational complexity of HHT filter, $m_f$ is a number of features selected, $F$ is a computational complexity for extracting a particular feature, $C_{CSP}$ is a computational complexity of using TRCSP filter. Thus Equation (1) gives a measure of the cognitive score for a particular algorithm and Equation (2) gives a measure of the computational complexity for the particular algorithm.

Table 2 provides the cognitive score of the 10 participants (CS) for executing cognitive tasks of a low cognitive load (L) and a high cognitive load (H), following Path1 through Path6. Since a result should clearly differentiate the high cognitive load (H) from the low cognitive load (L), achieving maximum separation between the high cognitive load (H) and the low cognitive load (L) is the purpose of this invention. As shown in Table 1, particularly the algorithm approach provided in path6 is disclosed in the present disclosure. As shown in path6, 2→3→4→6→8, the system 102 receives the EEG signals from a set of EEG channels associated with a left-frontal brain lobe (AF3, F7, F3, FC5). Further, system 102 preprocesses the EEG signals using a Hilbert-Huang Transform (HHT) filter to remove a noise corresponding to one or more non-cerebral artifacts to generate preprocessed EEG signals. Further, as shown in path6, the system 102 extracts only the features comprising Fast Fourier Transform (FFT) based alpha and theta band power from the preprocessed EEG signals. Further, as shown in path 6, system 102 generates a feature vector from the features comprising Fast Fourier Transform (FFT) based alpha and theta band power and classifies the feature vector using a SVM classifier to calculate the cognitive score (*CS*) and computational complexity (CC). Table 2 contains Cognitive score (CS) of the low cognitive load (L) and high cognitive load (H) tasks for 10 participants using path1 to path6.

Referring to Equation (2), the results of the Computational Complexity (CC) are discussed below. For path1, path3 and path5, value of $n_c$ = 14, further in Path2, path 4 and path6, value of $n_c$ =4. Hence by using less number of EEG channels, the Computational Complexity decreases for path2, path4 and path6. Initially for path1 and path2, number of features used are $m_f$ = 11. Hence higher CC for path 1 and path2. Further for path 3 to path6, the number of features used is $m_f$ = 2, hence reduced computational complexity for path 2 to path 6.

In path6, the computational complexity is further reduced by eliminating use of to TRCSP spatial filter. Hence path6 provides the least computational complexity in the experimental path1 to path6. The least computational complexity of path6 is attributed by i) Probing only left-frontal brain (AF3, F7, F3, FC5) ii) Using only alpha and theta band power features and iii) Alpha and theta band power features fed to SVM by eliminating use of TRCSP spatial filter. Since $m_f$ and $n_c$ of Equation (2) is reduced, so third component of equation (2) is also reduced to (2x3x $C_{CSP}$) compared to original one in path1 (14 x 11 x $C_{CSP}$).

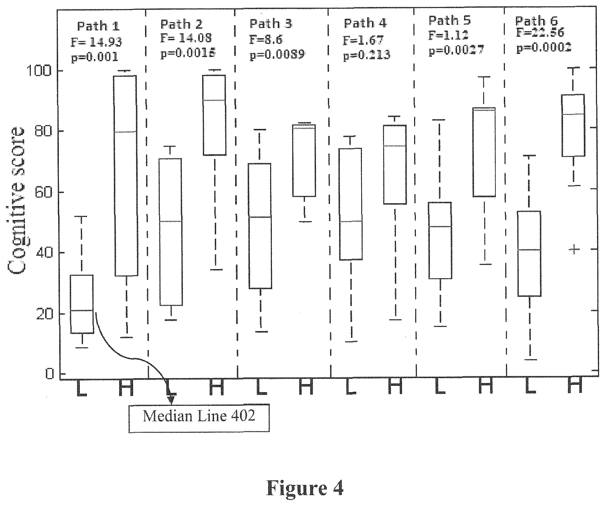

Figure 4 gives one-way Analysis of Variance (ANOVA) (Box plot) of results shown in Table 2. As shown in the Table 2, maximum separation is marked in bold. Results for $CS_{High} < CS_{Low}$ are italicized and underlined. In statistics ANOVA is a technique used to compare two or more groups of data.

$$F = \frac{\text{between-group variability}}{\text{within-group variability}}.$$

A higher value of F implies that samples are drawn from populations with different mean values, indicating that the samples belong to different groups. P is a probability of an observed result to be correct. The Box plot in Figure 4 and Table 2 shows that Path1 gives maximum separation between $CS_{High}$ and $CS_{Low}$ for 6 subjects. Whereas Path6 gives maximum separation between $CS_{High}$ and $CS_{Low}$ for only 1 subject. Further, Table 2 results also indicate that Path1gives maximum intra-class variance for $CS_{High}$ which is undesirable. While Path6 gives highest difference between mean $CS_{High}$ and mean $CS_{Low}$ while having minimal intra-class variance. Further, referring to Table 2, Path4 shows reverse trend for CS values for 3 subjects. Path1 also shows the reverse trend for 1 subject. The reverse trend shown for CS values is undesirable. It should be noted that Path1 and Path4 have used TRCSP filter while processing the EEG signals. Use of TRCSP filter in the EEG signals processing results in increased computational complexity (CC). Hence Path1 to Path 5, are not suitable/preferred for the EEG signal processing. While, Path6 offers maximum F-value and minimum p-value. Hence, the experimental results prove that Path6 is the most preferred path. Further, it is also proved that use of TRCSP spatial filter is not required to achieve accuracy and efficiency in processing of the EEG signals.

In a box plot, a median line is required to be at a middle of a box depicting ideal dispersion of data. Hence figure 4 shows, variation in position of the median line 402 for the box of Path1 to the box of Path6. Figure 4 shows that the boxes (H and L) of Path6 show the median line 402 is close to middle of the boxes. Results of Path3 and Path6 show that the median line 301 is at extreme top of the boxes, hence these results of Path3 and Path6 are not desirable. Further, there should be a clear separation in the low cognitive load (L) and low cognitive load (H) boxes of the box plot. The results of Path6 satisfy this box separation criterion. The results for Path6 show that there is clear separation in the H and L boxes of the box plot. While results of Path3 and Path4 shows that the H and L boxes of Path3 and Path4 are overlapping with each other. This overlapping of the H and L boxes is undesirable condition. Hence Path 1 to Path5 are not preferred and Path6 is the most preferred path.

Table 2: Cognitive score results of low cognitive load (L) and high cognitive load (H) tasks for 10 participants through path1 to path6

| Subjects | Path 1 | | Path 2 | | Path 3 | | Path 4 | | Path 5 | | Path 6 | |
|---|---|---|---|---|---|---|---|---|---|---|---|---|
| | CS (H) | CS (L) | CS (H) | CS (L) | CS (H) | CS (L) | CS (H) | CS (L) | CS (H) | CS (L) | CS (H) | CS (L) |
| 1 | 79.8 | 8.7 | 100 | 75 | 58.3 | 54.2 | 29 | 43 | 56 | 50 | **100** | **4** |
| 2 | **97.4** | **15.2** | 89.7 | 20.4 | 82.1 | 23.5 | 74.3 | 10.2 | 85.2 | 15.2 | 84.6 | 18.9 |
| 3 | 12 | 8.8 | **34.2** | **17.6** | 82.2 | 76.1 | 79 | 73 | 58 | 44 | 61 | 50 |
| 4 | **84** | **21** | 96.3 | 71.4 | 81.1 | 80.2 | 64.2 | 50 | 89 | 83 | 89 | 71 |
| 5 | **100** | **44** | 98 | 51.7 | 74.5 | 51.4 | 65 | 78 | 86 | 55 | 74 | 51 |
| 6 | **71.4** | **29** | 89.8 | 70.3 | 81.6 | 66.6 | 83 | 62 | 97 | 48 | 89 | 40 |
| 7 | 33.3 | 52 | **77.7** | **23.5** | 50 | 29.4 | 80.5 | 76.4 | 86 | 58 | 75 | 58 |
| 8 | **100** | **23.3** | 98.4 | 25.3 | 80.4 | 13.6 | 84 | 25.2 | 86 | 26.5 | 96 | 30.2 |
| 9 | 28.5 | 20.1 | 54.2 | 50.1 | **57.5** | **42.3** | 17.5 | 41.1 | 35.2 | 32.1 | 40.2 | 26.8 |
| 10 | **67.3** | **24.6** | 82.3 | 45 | 71.9 | 48.5 | 64.1 | 50.9 | 75.4 | 45.7 | 78.5 | 38.8 |

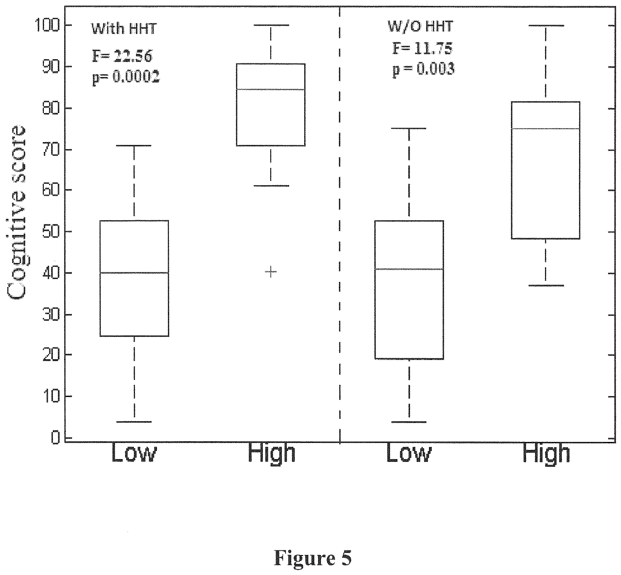

Referring to Figure 5, investigation of usefulness of Hilbert-Huang Transform (HHT) filter in processing of the EEG signals to determine the cognitive load of the subject (participant) is described. Figure 5 shows the cognitive score (CS) for processing of the EEG signals using the HHT filters or without using the HHT filters. Figure 4 shows effects of artifacts removal on determination of the Cognitive load (Cognitive score) from the EEG signals using Hilbert-Huang Transform (HHT) filter and without using HHT filter. The separation between cognitive score for high cognitive load (H) and low cognitive load (L) is greater and intra-class variance for H and L is lowest for artifact removed signals using HHT. Further maximum F-value is obtained for processing of the EEG signals using HHT filters.

The Exemplary embodiments discussed above may provide certain advantages. Though not required to practice aspects of the disclosure, these advantages may include those provided by the following features.

Some embodiments enable a system and a method for determining a cognitive load of a subject from Electroencephalography (EEG) signals received from low cost and low resolution EEG devices comprising a maximum of fourteen EEG channels.

Some embodiments enable the system and the method for determining the cognitive load of the subject using EEG signals received only from four EEG channels associated with the left-frontal brain lobe.

Some embodiments enable the system and the method for determining the cognitive load of the subject using EEG signals by using optimized method having minimum algorithmic complexity, by using minimum number of FFT based features to accurately determine the cognitive load.

Some embodiments enable the system and the method for eliminating noise from the EEG signals using HHT.

Some embodiments enable the system and the method for eliminating use of spatial filters in processing of the EEG signals with improved accuracy while determining the cognitive load of the subject.

Some embodiments enable the system and the method for reducing computational complexity by using of reduced number of EEG channels, reduced number of EEG features and algorithmic simplicity.

Some embodiments enable the system and the method for determining the cognitive load of the subject from EEG signals with superior accuracy using low resolution EEG devices.

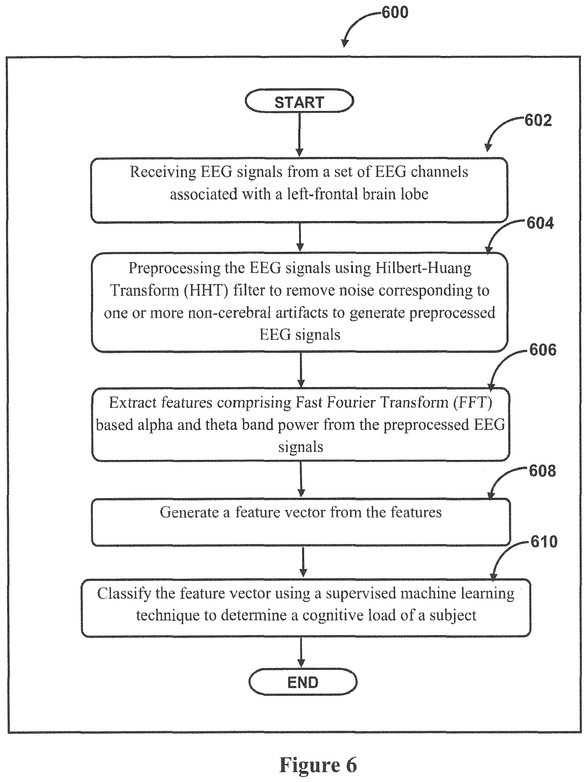

Referring now to Figure 6, a method 600 for determining a cognitive load of a subject from Electroencephalography (EEG) signals is shown, in accordance with an embodiment of the present subject matter. The method 600 may be described in the general context of computer executable instructions. Generally, computer executable instructions can include routines, programs, objects, components, data structures, procedures, modules, functions, etc., that perform particular functions or implement particular abstract data types. The method 600 may also be practiced in a distributed computing environment where functions are performed by remote processing devices that are linked through a communications network. In a distributed computing environment, computer executable instructions may be located in both local and remote computer storage media, including memory storage devices.

The method 600 can be implemented in any suitable hardware, software, firmware, or combination thereof. However, for ease of explanation, in the embodiments described below, the method 600 may be considered to be implemented in the above described system 102.

At block 602, the EEG signals are received. The EEG signals are received from a set of EEG channels associated with a left-frontal brain lobe. The EEG signals are associated with the subject performing a cognitive task. The EEG signals are received from a low resolution EEG device comprising a maximum of fourteen EEG channels. The set of EEG channels may comprise four EEG channels associated with the left-frontal brain lobe.

At block 604, the EEG signals are preprocessed to remove a noise corresponding to one or more non-cerebral artifacts to generate preprocessed EEG signals. The EEG signals are preprocessed using a Hilbert-Huang Transform (HHT) filter to remove the noise corresponding to the one or more non-cerebral artifacts to generate preprocessed EEG signals. The one or more non-cerebral artifacts may comprise artifacts not directly associated with brain activity and may be associated with other body parts or a machine.

At block 606, features are extracted from the preprocessed EEG signals. The features comprise Fast Fourier Transform (FFT) based alpha and theta band power based features.

At block 608, a feature vector is generated from the features.

At block 610, the feature vector is classified using a supervised machine learning technique to determine the cognitive load of the subject. The supervised machine learning technique may be a Support Vector Machine (SVM) classifier.

Although implementations of methods and systems for determining a cognitive load of a subject from Electroencephalography (EEG) signals have been described in a language specific to structural features and/or methods, it is to be understood that the appended claims are not necessarily limited to the specific features or methods described. Rather, the specific features and methods are disclosed as examples of implementations for determining the cognitive load of the subject from the EEG signals.

**Claims**

1. A method for determining a cognitive load of a subject from Electroencephalography (EEG) signals, the method comprising:

   receiving from a low resolution EEG device (120), by a processor (110), the EEG signals from a set of EEG channels associated with a left-frontal brain lobe, wherein the EEG signals are associated with the subject performing a cognitive task, wherein the low resolution EEG device (120) is linked with a maximum of fourteen EEG channels;
   the method being **characterized by** the further steps of:

   preprocessing, by the processor (110), the EEG signals using a Hilbert-Huang Transform (HHT) filter that decomposes the EEG signals into empirical mode signals and remove a noise corresponding to one or more non-cerebral artifacts by replacing frequency of the EEG signals with a median value at a time point when the frequency of the EEG signals overshoots a predefined level at the time point, to generate preprocessed EEG signals, wherein the non-cerebral artifacts are originated from body parts and outside body parts, movement of the subject, settling of EEG electrodes, spikes originating from a momentary change in an impedance of the EEG electrode;
   extracting, by the processor (110), features comprising Fast Fourier Transform (FFT) based alpha and theta band power from the preprocessed EEG signals;
   generating, by the processor (110), a feature vector from the features; and
   classifying, by the processor, the feature vector using a supervised machine learning technique to determine the cognitive load of the subject.

2. The method of claim 1, wherein the set of EEG channels comprises four EEG channels associated with the left-frontal brain lobe.

3. The method of claim 1, wherein the supervised machine learning technique is a Support Vector Machine (SVM) classifier.

4. A system for determining a cognitive load of a subject from Electroencephalography (EEG) signals, the system comprising:

a processor (110);
a low resolution EEG device (120);
a memory (114) coupled to the processor (110), wherein the processor (110) is configured to:

receive, from the low resolution EEG device (120), the EEG signals from a set of EEG channels associated with a left-frontal brain lobe, wherein the EEG signals are associated with the subject performing a cognitive task, wherein the low resolution EEG device (120) is linked with a maximum of fourteen EEG channels; **characterized in that** the processor (110) is further configured to:

preprocess the EEG signals using a Hilbert-Huang Transform (HHT) filter that decomposes the EEG signals into empirical mode signals and remove a noise corresponding to one or more non-cerebral artifacts by replacing frequency of the EEG signals with a median value at a time point when the frequency of the EEG signals overshoots a predefined level at the time point, to generate preprocessed EEG signals, wherein the non-cerebral artifacts are originated from body parts and outside body parts, movement of the subject, settling of EEG electrodes, spikes originating from a momentary change in an impedance of the EEG electrode;
extract features comprising Fast Fourier Transform (FFT) based alpha and theta band power from the preprocessed EEG signals;
generate a feature vector from the features; and
classify the feature vector using a supervised machine learning technique to determine the cognitive load of the subject.

5. The system of claim 4, wherein the set of EEG channels comprises four EEG channels associated with the left-frontal brain lobe.

6. The system of claim 4, wherein the supervised machine learning technique is a Support Vector Machine (SVM) classifier.

**Patentansprüche**

1. Verfahren zum Bestimmen einer kognitiven Belastung eines Subjekts aus Elektroenzephalographie(EEG)-Signalen, wobei das Verfahren Folgendes umfasst:

Empfangen, von einer niedrigauflösenden EEG-Vorrichtung (120) durch einen Prozessor (110), der EEG-Signale aus einem Satz von EEG-Kanälen, die einem linken Frontallappen zugehörig sind, wobei die EEG-Signale dem Subjekt zugehörig sind, das eine kognitive Aufgabe durchführt, wobei die niedrigauflösende EEG-Vorrichtung (120) mit maximal vierzehn EEG-Kanälen verknüpft ist;
wobei das Verfahren **gekennzeichnet ist durch** die folgenden weiteren Schritte:

Vorverarbeiten, durch den Prozessor (110), der EEG-Signale unter Verwendung eines Hilbert-Huang-Transformations(HHT)-Filters, das die EEG-Signale in empirische Modussignale zerlegt und ein Rauschen, das einem oder mehreren nicht-zerebralen Artefakten entspricht, durch Ersetzen einer Frequenz der EEG-Signale mit einem Medianwert zu einem Zeitpunkt, zu dem die Frequenz der EEG-Signale einen zuvor definierten Pegel zu dem Zeitpunkt überschreitet, entfernt, um vorverarbeitete EEG-Signale zu erzeugen, wobei die nicht-zerebralen Artefakte aus Körperteilen und äußeren Körperteilen, einer Bewegung des Subjekts, einem Absetzen von EEG-Elektroden, aus einer momentanen Änderung einer Impedanz der EEG-Elektrode stammenden Spitzen stammen;
Extrahieren, durch den Prozessor (110), von Merkmalen, die eine auf einer Fast-Fourier-Transformation (FFT) basierende Alpha- und Theta-Bandleistung umfassen, aus den vorverarbeiteten EEG-Signalen;
Erzeugen, durch den Prozessor (110), eines Merkmalsvektors aus den Merkmalen; und
Klassifizieren, durch den Prozessor, des Merkmalsvektors unter Verwendung einer überwachten Maschinenlerntechnik, um die kognitive Belastung des Subjekts zu bestimmen.

**2.** Verfahren nach Anspruch 1, wobei der Satz von EEG-Kanälen vier EEG-Kanäle umfasst, die dem linken Frontallappen zugehörig sind.

**3.** Verfahren nach Anspruch 1, wobei die überwachte Maschinenlerntechnik ein Stützvektormaschinen(SVM)-Klassifizierer ist.

**4.** System zum Bestimmen einer kognitiven Belastung eines Subjekts aus Elektroenzephalographie(EEG)-Signalen, wobei das System Folgendes umfasst:

einen Prozessor (110);
eine niedrigauflösende EEG-Vorrichtung (120);
einen Speicher (114), der mit dem Prozessor (110) gekoppelt ist, wobei der Prozessor (110) zu Folgendem konfiguriert ist:

Empfangen, von der niedrigauflösenden EEG-Vorrichtung (120), der EEG-Signale aus einem Satz von EEG-Kanälen, die einem linken Frontallappen zugehörig sind, wobei die EEG-Signale dem Subjekt zugehörig sind, das eine kognitive Aufgabe durchführt, wobei die niedrigauflösende EEG-Vorrichtung (120) mit maximal vierzehn EEG-Kanälen verknüpft ist;
**dadurch gekennzeichnet, dass** der Prozessor (110) ferner zu Folgendem konfiguriert ist:

Vorverarbeiten der EEG-Signale unter Verwendung eines Hilbert-Huang-Transformations(HHT)-Filters, das die EEG-Signale in empirische Modussignale zerlegt und ein Rauschen, das einem oder mehreren nicht-zerebralen Artefakten entspricht, durch Ersetzen einer Frequenz der EEG-Signale mit einem Medianwert zu einem Zeitpunkt, zu dem die Frequenz der EEG-Signale einen zuvor definierten Pegel zu dem Zeitpunkt überschreitet, entfernt, um vorverarbeitete EEG-Signale zu erzeugen, wobei die nicht-zerebralen Artefakte aus Körperteilen und äußeren Körperteilen, der Bewegung des Subjekts, dem Absetzen von EEG-Elektroden, aus einer momentanen Änderung einer Impedanz der EEG-Elektrode stammenden Spitzen stammen;
Extrahieren von Merkmalen, die eine auf der Fast-Fourier-Transformation (FFT) basierende Alpha- und Theta-Bandleistung umfassen, aus den vorverarbeiteten EEG-Signalen;
Erzeugen eines Merkmalsvektors aus den Merkmalen; und
Klassifizieren des Merkmalsvektors unter Verwendung einer überwachten Maschinenlerntechnik, um die kognitive Belastung des Subjekts zu bestimmen.

**5.** System nach Anspruch 4, wobei der Satz von EEG-Kanälen vier EEG-Kanäle umfasst, die dem linken Frontallappen zugehörig sind.

**6.** System nach Anspruch 4, wobei die überwachte Maschinenlerntechnik ein Stützvektormaschinen(SVM)-Klassifizierer ist.

**Revendications**

**1.** Procédé destiné à déterminer une charge cognitive d'un sujet à partir de signaux d'électroencéphalographie (EEG), le procédé comprenant :

la réception d'un dispositif EEG à basse résolution (120), par un processeur (110), des signaux EEG d'un ensemble de canaux EEG associés à un lobe frontal gauche, les signaux EEG étant associés au sujet effectuant une tâche cognitive, le dispositif EEG à basse résolution (120) étant lié à un maximum de quatorze canaux EEG ;
le procédé étant **caractérisé par** les étapes supplémentaires suivantes :

le prétraitement, par le processeur (110), des signaux EEG à l'aide d'un filtre de transformée de Hilbert-Huang (HHT) qui décompose les signaux EEG en signaux de mode empirique et supprime un bruit correspondant à un ou plusieurs artefacts non cérébraux en remplaçant la fréquence des signaux EGG avec une valeur médiane à un point temporel lorsque la fréquence des signaux EEG dépasse un niveau prédéfini au point temporel, pour générer des signaux EEG prétraités, les artefacts non cérébraux provenant de parties du corps et de parties du corps extérieures, du mouvement du sujet, du réglage des électrodes EEG, des pointes provenant d'un changement momentané d'une impédance de l'électrode EEG;

l'extraction, par le processeur (110), de caractéristiques comprenant une puissance de bande alpha et thêta basée sur la transformée de Fourier rapide (FFT) à partir des signaux EEG prétraités ;

la génération, par le processeur (110), d'un vecteur de caractéristiques à partir des caractéristiques ; et

le classement, par le processeur, du vecteur de caractéristiques à l'aide d'une technique d'apprentissage automatique supervisé pour déterminer la charge cognitive du sujet.

2. Procédé selon la revendication 1, dans lequel l'ensemble de canaux EEG comprend quatre canaux EEG associés au lobe frontal gauche.

3. Procédé selon la revendication 1, dans lequel la technique d'apprentissage automatique supervisé est un classificateur de machine à vecteurs de support (SVM).

4. Système de détermination d'une charge cognitive d'un sujet à partir de signaux d'électroencéphalographie (EEG), le système comprenant :

un processeur (110) ;

un dispositif EEG à basse résolution (120) ;

une mémoire (114) couplée au processeur (110), le processeur (110) étant configuré pour:

recevoir, à partir du dispositif EEG à basse résolution (120), les signaux EEG d'un ensemble de canaux EEG associés à un lobe cérébral frontal gauche, les signaux EEG étant associés au sujet effectuant une tâche cognitive, le dispositif EEG à basse résolution (120) étant lié à un maximum de quatorze canaux EEG ;

**caractérisé en ce que** le processeur (110) est en outre configuré pour :

prétraiter les signaux EEG à l'aide d'un filtre de transformée de Hilbert-Huang (HHT) qui décompose les signaux EEG en signaux de mode empirique et supprime un bruit correspondant à un ou plusieurs artefacts non cérébraux en remplaçant la fréquence des signaux EEG par une valeur médiane à un point temporel lorsque la fréquence des signaux EEG dépasse un niveau prédéfini au point temporel, pour générer des signaux EEG prétraités, les artefacts non cérébraux provenant de parties du corps et de parties extérieures du corps, du mouvement du sujet, du réglage des électrodes EEG, des pointes provenant d'un changement momentané d'une impédance de l'électrode EEG ;

extraire des caractéristiques comprenant la puissance des bandes alpha et thêta basées sur la transformée de Fourier rapide (FFT) à partir des signaux EEG prétraités ;

générer un vecteur de caractéristiques à partir des caractéristiques ; et

classer le vecteur de caractéristiques à l'aide d'une technique d'apprentissage automatique supervisé pour déterminer la charge cognitive du sujet.

5. Système selon la revendication 4, dans lequel l'ensemble de canaux EEG comprend quatre canaux EEG associés au lobe frontal gauche.

6. Système selon la revendication 4, dans lequel la technique d'apprentissage automatique supervisé est un classificateur de machine à vecteurs de support (SVM).

100

SYSTEM (102)

PROCESSOR(S) (110)

I/O INTERFACE(S) (112)

MEMORY (114)

DATA (116)

DATABASE (118)

Electroencephalography (EEG) device (120)

NETWORK (106)

104 - 1

104 - 2

104 - 3

104 - N

ELECTRONIC DEVICES (104)

Figure 1

15

**Figure 2**

Figure 3

**Figure 4**

**Figure 5**

—600

```
                          START                     ┌─602

          ┌─────────────────────────────────────────┐
          │  Receiving EEG signals from a set of EEG channels  │
          │       associated with a left-frontal brain lobe    │
          └─────────────────────────────────────────┘
                                                    ┌─604
          ┌─────────────────────────────────────────┐
          │  Preprocessing the EEG signals using Hilbert-Huang │
          │  Transform (HHT) filter to remove noise corresponding to │
          │  one or more non-cerebral artifacts to generate preprocessed │
          │                    EEG signals               │
          └─────────────────────────────────────────┘
                                                    ┌─606
          ┌─────────────────────────────────────────┐
          │  Extract features comprising Fast Fourier Transform (FFT) │
          │  based alpha and theta band power from the preprocessed EEG │
          │                     signals                  │
          └─────────────────────────────────────────┘
                                                    ┌─608
          ┌─────────────────────────────────────────┐
          │      Generate a feature vector from the features    │
          └─────────────────────────────────────────┘
                                                    ┌─610
          ┌─────────────────────────────────────────┐
          │  Classify the feature vector using a supervised machine learning │
          │   technique to determine a cognitive load of a subject  │
          └─────────────────────────────────────────┘

                           END
```

**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 3390MUM2014
- US 2007185697 A1
- CN 102063180 B
- EP 2200347 A2

**Non-patent literature cited in the description**

- **BESSERVE, M. et al.** Prediction of performance level during a cognitive task from ongoing EEG oscillatory activities. *Clinical Neurophysiology,* vol. 119 (4), 897-908
- **GEVINS, A.** High-resolution EEG mapping of cortical activation related to working memory: effects of task difficulty, type of processing, and practice. *Cerebral Cortex,* vol. 7 (4), 374-385
- **CHATTERJEE DEBATRI et al.** EEG-based fuzzy cognitive load classification during logical analysis of program segments. *IEEE INTERNATIONAL CONFERENCE ON FUZZY SYSTEMS (FUZZ-IEEE),* 2013